# EUROPEAN PATENT APPLICATION

(11) **EP 4 834 945 A1**
(43) Date of publication of application: **04.11.2026**
(21) Application number: 26174662.2
(22) Date of filing: 27.04.2026
(51) Int. Cl.: C07K 16/44

(54) **ANTIBODY OR ANTIGEN BINDING FRAGMENT THEREOF SPECIFIC FOR THE WHITLOW 218 LINKER**

(30) Priority: 29.04.2025 EP 25173346
(71) Applicant: Miltenyi Biotec B.V. & Co. KG, 51429 Bergisch Gladbach (DE)
(72) Inventor: WINKELS, Monika, 51429 Bergisch Gladbach (DE); FELSBERGER, André, 51429 Bergisch Gladbach (DE); DROSTE, Miriam, 51429 Bergisch Gladbach (DE); WILMES, Stephan, 51429 Bergisch Gladbach (DE)
(74) Representative: Biervert, Christian

(57) **Abstract**

The present invention provides an antibody or antigen binding fragment thereof specific for the amino acid sequence set forth in SEQ ID NO:1 (the whitlow linker sequence), wherein said antibody or antigen binding fragment thereof comprises a) a heavy chain variable region of an antibody (VH) comprising SEQ ID NO:2, and b) a light chain variable region of an antibody (VL) comprising SEQ ID NO:3.

## Description

### Field of the invention

The present invention generally relates to the field of in-vitro detection, analysis and /or separation of immune cells that express a chimeric antigen receptor (CAR) by using antibodies or antigen binding fragments thereof that recognize an extracellular part of the CAR, in particular to the field of in-vitro detection, analysis and /or separation of immune cells that express a chimeric antigen receptor (CAR) comprising the Whitlow 218 linker by using antibodies or antigen binding fragments thereof that recognize said Whitlow 218 linker.

### Background of the invention

The use of chimeric antigen receptor (CAR)-expressing immune cells such as T cells re-directed to specifically recognize and eliminate target cells such as malignant cells, greatly increased the scope and potential of adoptive immunotherapy and is being assessed for new standard of care in certain human disorders such as malignancies. CARs are recombinant receptors that typically target surface molecules in a human leukocyte antigen (HLA)-independent manner. Generally, CARs comprise an extracellular antigen recognition moiety, often a single-chain variable fragment (scFv) derived from antibodies or a Fab fragment, linked to an extracellular spacer, a transmembrane domain and intracellular co-stimulatory and signaling domains. Identification of CAR-positive cells is very important for several applications. Common applications include in-process control (IPC), quality control (QC), immune-monitoring, concomitant research and further analytics. High-quality detection reagents that enable specific binding of CAR-positive cells are essential for this. These detection reagents often have to be manufactured individually, which is time-consuming and expensive. This is often not possible, especially in academia, where the final CAR sequence is often not yet defined. One idea to circumvent this issue is to use sequences for antibody detection that are frequently used in CARs. Promising structures for this purpose are the linker sequences within CAR single chain variable fragments (scFv), which connect the heavy and light chains in the scFv. Currently, two linker sequences are predominantly used in commercial CAR targets: the Whitlow 218 linker (also herein referred to as Whitlow linker) and the G4S linker. The performance of a commercial Whitlow linker-targeting antibody for detecting the frequency of CAR T cells was recently compared to a specific antigen-based CAR detection reagent. Grahnert et al. (Biomedicines 2024, 12, 1641) demonstrated that the Whitlow linker-targeting antibody did not meet the performance of the CD19 CAR-specific detection reagent. Therefore, although antibodies or antigen binding fragments thereof that specifically bind to the Whitlow linker exist, there is a need in the art for improved and or alternative antibodies or antigen binding fragments thereof that specifically bind to the Whitlow linker.

### Brief description of the invention

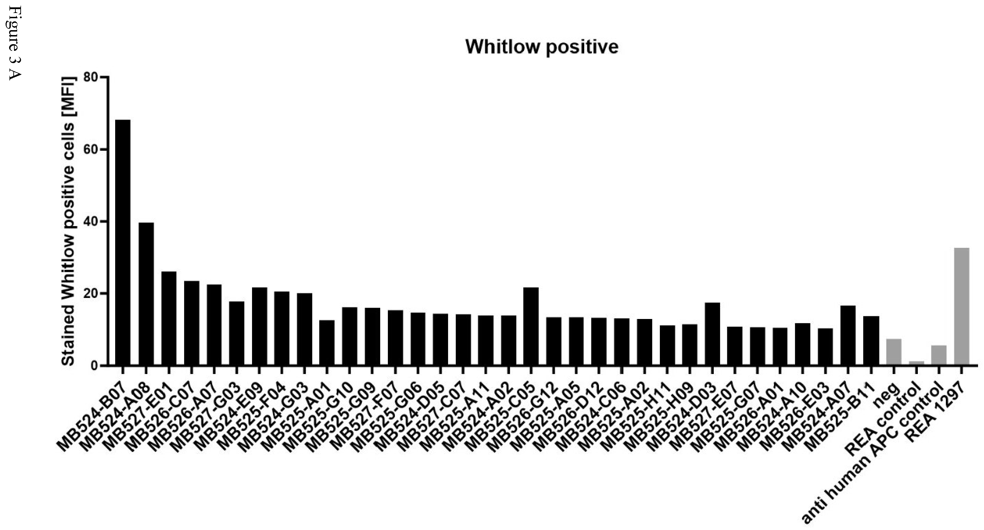

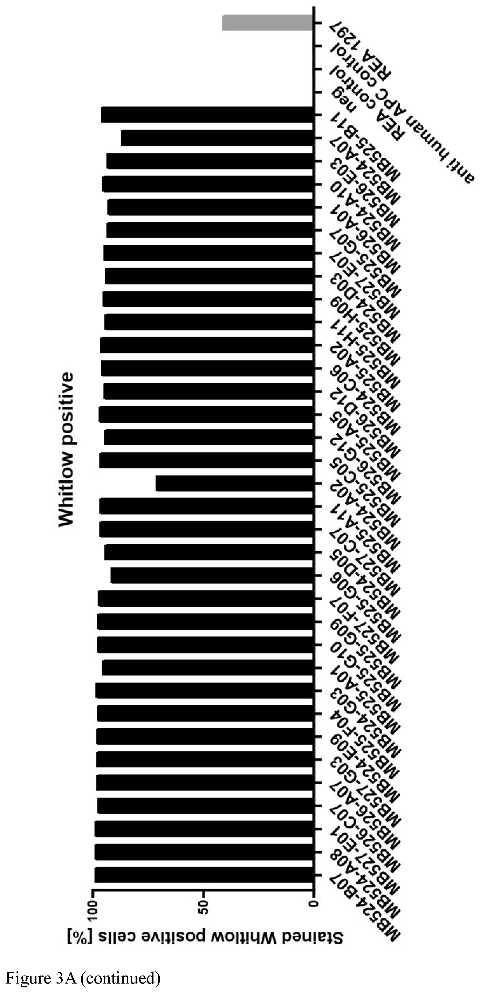

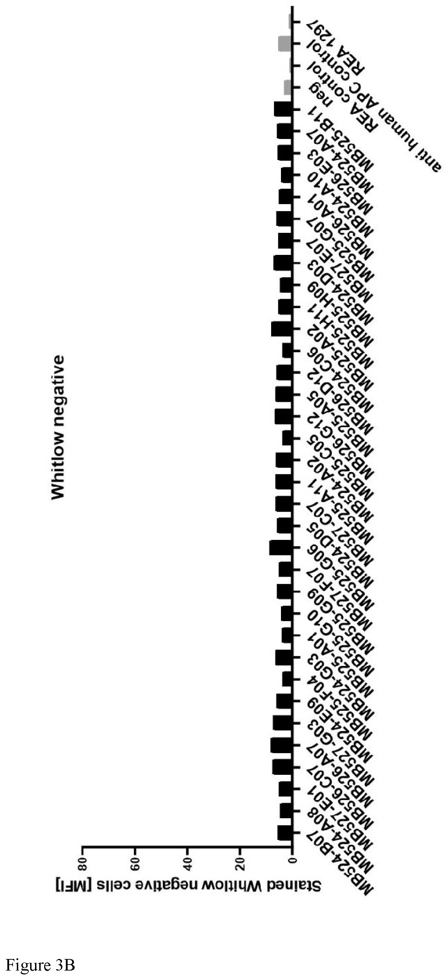

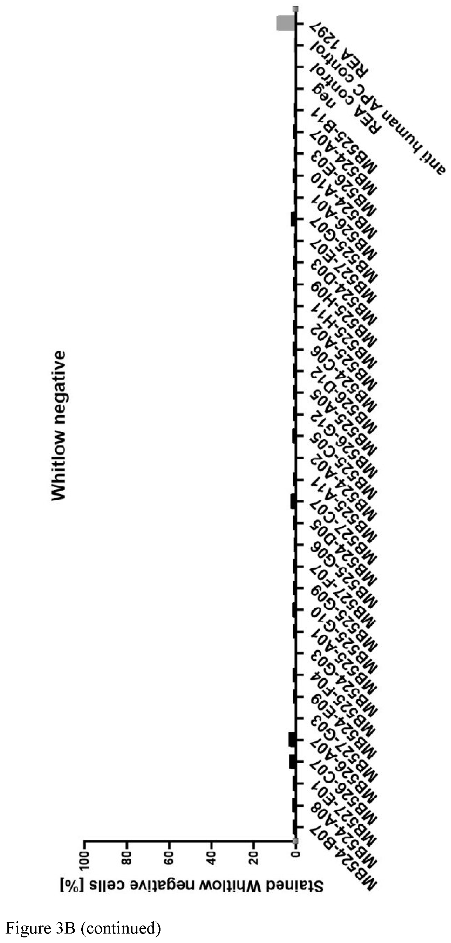

The inventors surprisingly found that an antibody or antigen binding fragment thereof comprising a) a heavy chain variable region of an antibody (VH) comprising SEQ ID NO:2, and b) a light chain variable region of an antibody (VL) comprising SEQ ID NO:3, can specifically bind to the amino acid sequence set forth in SEQ ID NO:1 (i.e. the Whitlow linker sequence). The antibody or antigen binding fragment thereof comprising SEQ ID NO:2 and SEQ ID NO:3 shows a better performance as compared to antibodies or antigen binding fragments thereof comprising scFv sequences as presented in SEQ ID NO:5 to SEQ ID NO:37, respectively (see Figure 3 A and B ). This allows for in-vitro assay and applications including in-process control (IPC), quality control (QC), immune-monitoring, concomitant research and further analytics of CAR immune cells that express a CAR having the sequence of the Whitlow linker as a linker between the VH and VL of the antigen binding domain of the CAR..

### Brief description of the drawings

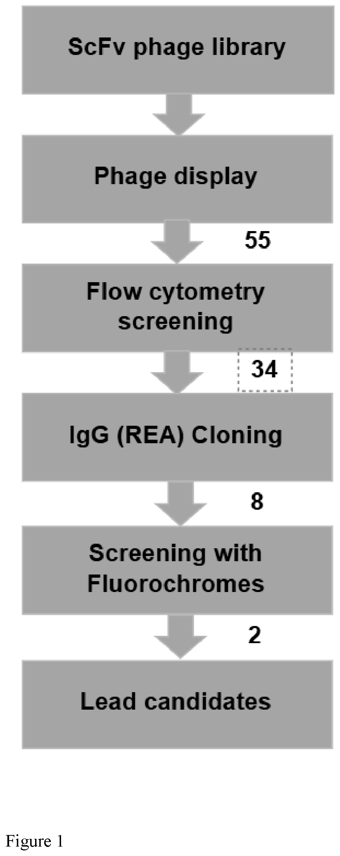

**Figure** 1: Overview of an identification and selection workflow for novel, fully human Whitlow-Linker binding domain candidates. 55 anti-Whitlow-Linker candidate binders were identified via phage display from a human antibody library. 34 candidates were further selected via flow cytometric screening of Whitlow-Linker expressing cells to select for Whitlow-Linker-specific candidates. Following, further *in vitro* analysis with fluorochrome coupled candidates allowed to identify two novel, functional and specific anti-Whitlow-Linker binders.

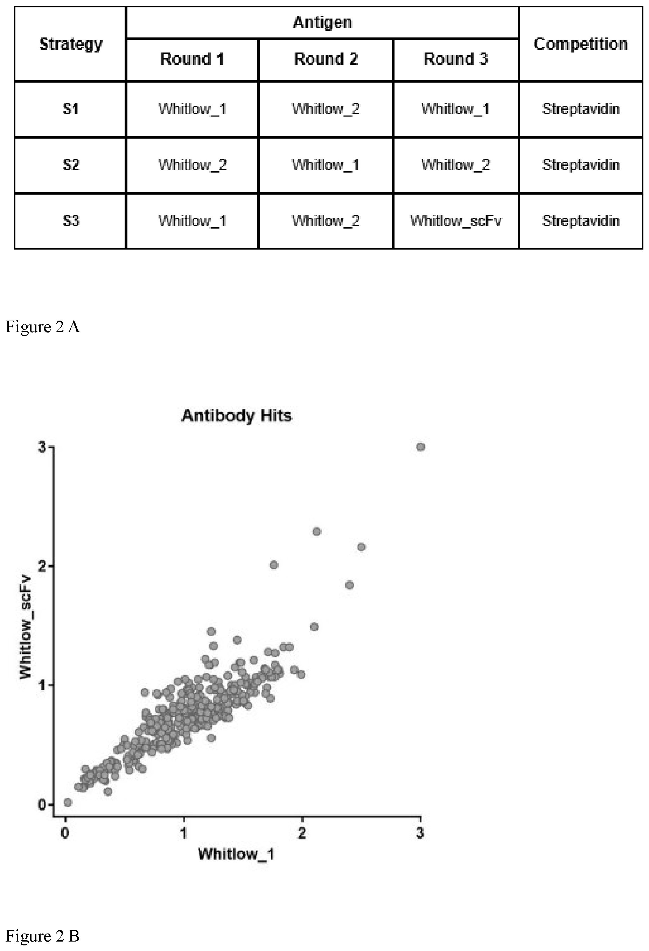

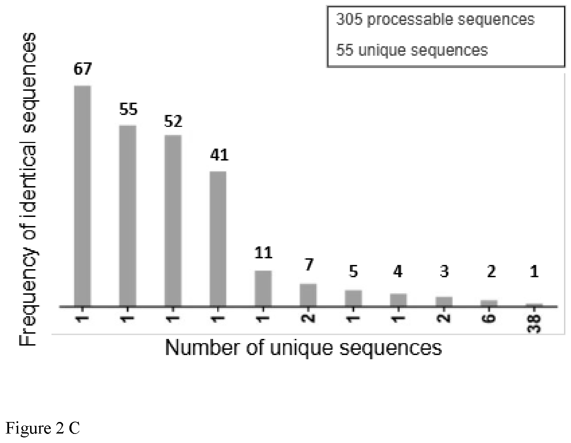

**Figure 2**: Unique binder sequences with reactivity on different Whitlow Peptide variants were identified via phage display. **A** Three different panning strategies were used to identify binder candidates using Whitlow_1 (Miltenyi Biotec) and Whitlow_2 (Miltenyi Biotec) in Strategy S1 - S2 as well as Whitlow_scFv (Miltenyi Biotec) in Strategy S3. In all strategies Streptavidin (VWR/Avantor) antigen was used for competition. **B** The used phage display antibody library revealed 322 first hits binding Whitlow peptide and scFv in ELISA. ELISA signals are displayed as absorbance values (450-620 nm). **C** Sequencing results confirm 55 unique sequences derived from 305 processable sequences.
**Figure 3****:** Flow cytometric screening revealed potential candidates in scFv-Fc format with high specificity for Whitlow-Linker. Candidates were produced in scFv-Fc format by transfection of HEK293 cells. CD2019CAR Sup-T1 cells were used to test for specificity of the candidates against Whitlow-Linker. 1x10⁵ cells were plated and stained with 100nM scFv-Fc for 30 minutes at 4°C. An anti-IgG(Fc)-APC secondary antibody staining (Jackson ImmunoResearch; Polyclonal, Cat. no. 109-136-098) was performed for 10 minutes at 4°C. Flow cytometric measurements are displayed as MFI and frequency (%) of stained cells. **A** Flow cytometric results of candidates in scFv-Fc format binding to Whitlow-Linker expressing Sup-T1 cells. **B** Flow cytometric results of candidates in scFv-Fc format binding to Whitlow-Linker non expressing SupT1 wt cells.
   REA control: REA Control Antibody (I), human IgG1 APC07 (Miltenyi Biotec, Cat. No. 130-120-709), anti-human APC control: Allophycocyanin (APC) AffiniPure F(ab')₂ Fragment Goat Anti-Human IgG, Fcy fragment specific (Jackson ImmunoResearch; Polyclonal, Cat. no. 109-136-098), REA1297: CD19 CAR FMC63 Idiotype Antibody, REAfinity^{™} (Miltenyi Biotec, Cat. No. 130-127-343).

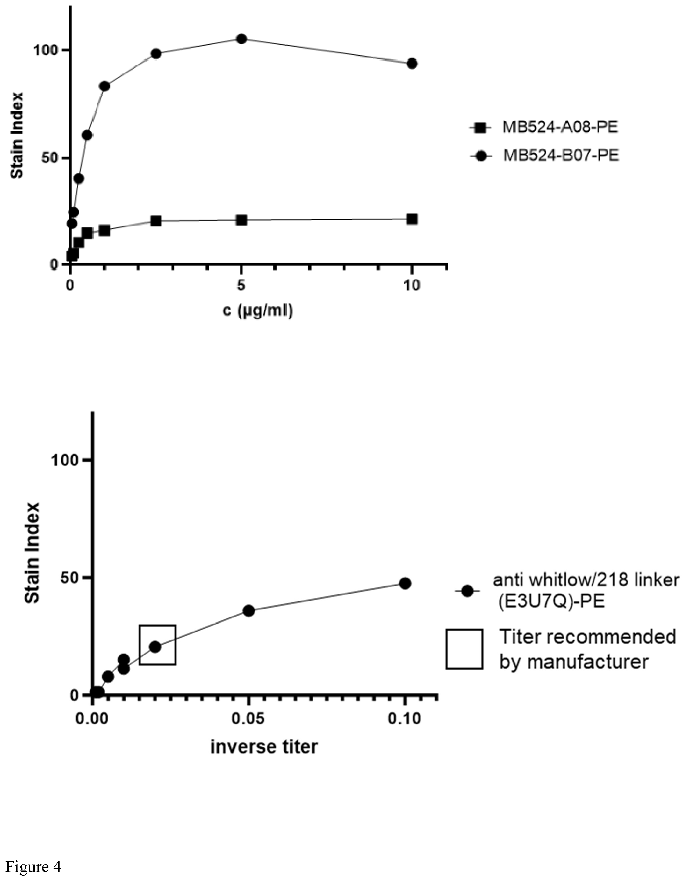

**Figure 4****:** Titration curve of different anti Whitlow antibody PE conjugates on CAR-Whitlow transduced SupT1 cells mixed with SupT1 wildtype cells. The diagrams show on the Y-axis the stain index of anti Whitlow+ cells using PE-conjugated anti Whitlow antibody conjugates. Top panel: The X-axis indicates the concentrations of the different anti Whitlow antibody PE reagents (0.05 µg/mL, 0.1 µg/mL, 0.2 µg/mL, 0.5 µg/mL, 2 µg/mL, 5 µg/mL, 10 µg/mL). Bottom panel: The X-axis indicates the inverse titers of a purchased anti Whitlow antibody PE (no concentration was provided). The data was obtained from one representative experiment.

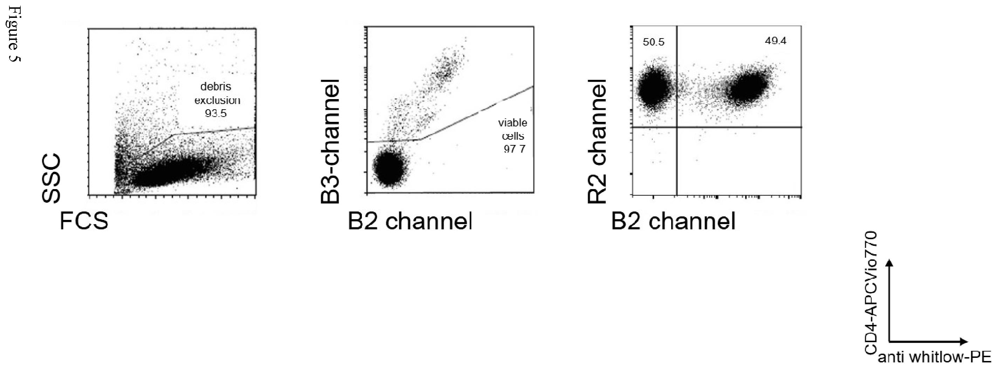

**Figure 5**: Example of flow cytometric data of anti Whitlow-PE staining on CAR-Whitlow transduced SupT1 cells mixed with SupT1 wildtype cells. The gating tree is as follows from left to right: first debris are excluded in the plot SSC/FSC, next viable cells are gated in the plot 7-AAD /anti Whitlow-PE. Finally in the plots CD4/ anti Whitlow-PE a quadrant is set to distinguish CAR+ (upper right) and CAR- (upper left) cells.

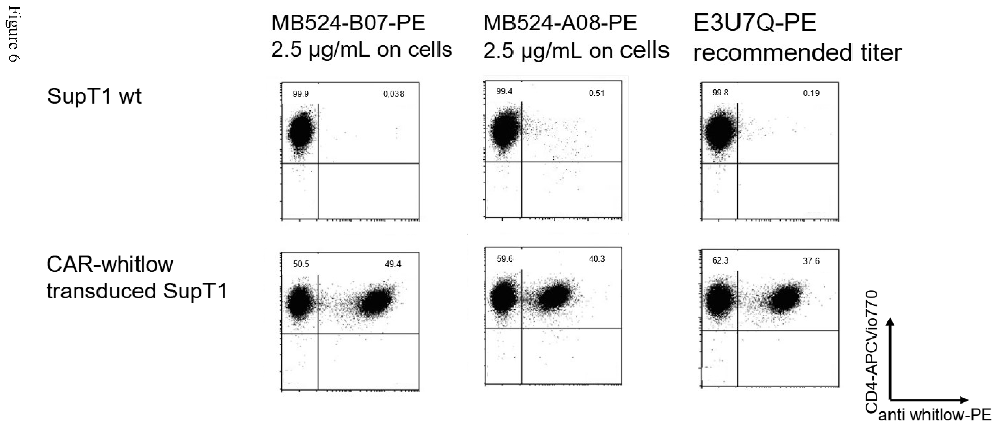

**Figure 6****:** For assessing the staining performance of different anti Whitlow antibodies, indicated clones were directly compared (clone MB524-B07, clone MB524-A08 and a commercial clone E3U7Q). CAR-Whitlow transduced SupT1 cells and SupT1 wildtype cells were stained separately with a mixture of CD4-APCVio770, 7-AAD and anti Whitlow-PE conjugates. After washing the cells were directly analyzed using the MACSQuant^{®}Analyzer10 using 100µL CAR-Whitlow transduced SupT1 cells mixed with 100µL SupT1 wildtype cells.

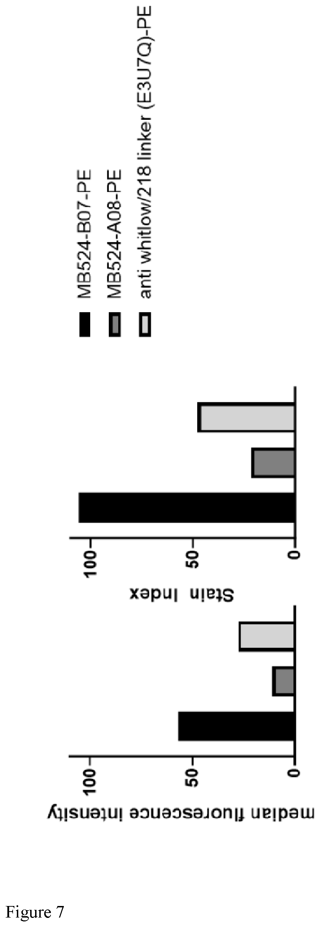

**Figure 7****:** Comparison of staining performance of anti Whitlow-PE clone MB524-B07 anti Whitlow-PE clone MB524-A08 and a commercially available anti Whitlow-PE conjugate clone E3U7Q (Cell Signaling Technology). Median fluorescence intensity and stain index of the conjugates in the optimal staining concentrations were measured on CAR-Whitlow transduced SupT1 cells mixed with SupT1 wildtype cells.

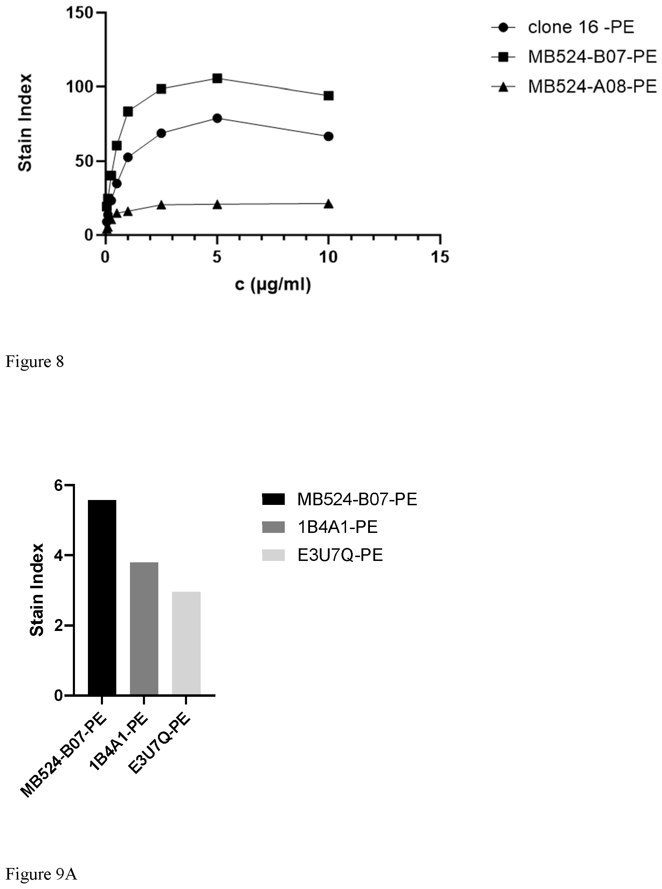

**Figure 8****:** Comparison of anti whitlow antibody PE conjugates on CAR-whitlow transduced SupT1 mixed with SupT1 wildtype cells -Titration curve
   The diagrams show on the Y-axis the stain index of anti Whitlow+ cells using PE-conjugated anti Whitlow antibody conjugates. The X-axis indicates the concentrations of the different anti Whitlow antibody PE reagents (0.05 µg/mL, 0.1 µg/mL, 0.2 µg/mL, 0.5 µg/mL, 2 µg/mL, 5 µg/mL, 10 µg/mL). Compared are staining performance of anti Whitlow-PE clone MB524-B07 anti Whitlow-PE clone MB524-A08 and an anti Whitlow-PE conjugate clone 16 that is a described anti Whitlow binder (clone 16 of US20200048681 having SEQ ID NO: 42 (VH) and SEQ ID NO:43 (VL))). The data was obtained from one representative experiment.

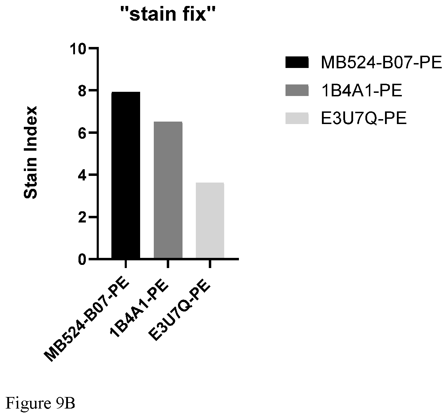

**Figure 9****: A** Comparison of staining performance of anti Whitlow-PE clone MB524-B07 with two commercially available anti Whitlow-PE conjugate clone E3U7Q and clone 1B4A1 (both from Cell Signaling Technology; clone 1B4A1 is also described in WO2025255276A1). Stain index of the conjugates in the optimal staining concentrations was measured on CAR-Whitlow transduced primary CAR T cells.

**B** Comparison of staining performance of anti Whitlow-PE clone MB524-B07 with two commercially available anti Whitlow-PE conjugate clone E3U7Q and clone 1B4A1 (both from Cell Signaling Technology). Stain index of the conjugates in the optimal staining concentrations was measured on stained and subsequently paraformaldehyde (PFA) fixed CAR-Whitlow transduced primary CAR T cells.

### Detailed description of the invention

In a first aspect the present invention provides an antibody or antigen binding fragment thereof specific for the amino acid sequence set forth in SEQ ID NO:1 (the Whitlow linker sequence), wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.

Said antibody or antigen binding fragment thereof, wherein said antigen binding fragment of said antibody is a Fab (fragment antigen binding), scFv (single chain fragment variable), Fab' or F(ab')2.

Said antibody or antigen binding fragment thereof, wherein said antibody or antigen binding fragment thereof is a scFv comprising SEQ ID NO:2 (VH) and SEQ ID NO:3 (VL).

Said antibody or antigen binding fragment thereof, wherein said antibody or antigen binding fragment thereof is a scFv comprising SEQ ID NO:2 (VH) and SEQ ID NO:3 (VL) in the order from N-terminus to C-terminus SEQ ID NO:2 (VH) and SEQ ID NO:3 (VL).

Said antibody or antigen binding fragment thereof, wherein said antibody or antigen binding fragment thereof is a scFv comprising SEQ ID NO:2 (VH) and SEQ ID NO:3 (VL) in the order from N-terminus to C-terminus SEQ ID NO:3 (VL) and SEQ ID NO:2 (VH).

Said antibody or antigen binding fragment thereof, wherein said antibody or antigen binding fragment thereof is a scFv comprising SEQ ID NO:4.

Said antibody or antigen binding fragment thereof, wherein said sequence of SEQ ID NO:1 to that said antibody or antigen binding fragment thereof binds is part of a fusion protein comprising said SEQ ID NO: 1.

Said antibody or antigen binding fragment thereof, wherein said sequence of SEQ ID NO:1 to that said antibody or antigen binding fragment thereof binds is part of a fusion protein comprising said SEQ ID NO:1 which connects two different protein domains.

Said antibody or antigen binding fragment thereof, wherein said sequence of SEQ ID NO:1 to that said antibody or antigen binding fragment thereof binds is part of an extracellular antigen binding domain of a chimeric antigen receptor expressed on the surface of an immune cell. Said antibody or antigen binding fragment thereof, wherein said sequence of SEQ ID NO:1 to that said antibody or antigen binding fragment thereof binds is part of an extracellular antigen binding domain of a chimeric antigen receptor expressed on the surface of an immune cell, wherein said antigen binding domain of said CAR is a scFv.

Said antibody or antigen binding fragment thereof, wherein said sequence of SEQ ID NO:1 to that said antibody or antigen binding fragment thereof binds is part of an extracellular antigen binding domain of a chimeric antigen receptor expressed on the surface of an immune cell, wherein said antigen binding domain of said CAR is a scFv, wherein antigen binding domain comprises SEQ ID NO:41.

Said antibody or antigen binding fragment thereof as disclosed herein, wherein said antibody or antigen binding fragment thereof is coupled to a detection moiety.

Said detection moiety may be a chromophore, a fluorescent moiety, a phosphorescent moiety, a luminescent moiety, a light absorbing moiety, a radioactive moiety, a transition metal isotope mass tag moiety, or a magnetic particle.

In another aspect the present invention provides a kit comprising an antibody or antigen binding fragment thereof, wherein said antibody or antigen binding fragment thereof is specific for the amino acid sequence set forth in SEQ ID NO:1 (the Whitlow linker sequence), wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3,
and wherein said antibody or antigen binding fragment thereof is coupled to a detection moiety.

Said detection moiety may be a chromophore, a fluorescent moiety, a phosphorescent moiety, a luminescent moiety, a light absorbing moiety, a radioactive moiety, a transition metal isotope mass tag moiety, or a magnetic particle.

Said kit, wherein said antibody or antigen binding fragment thereof is a scFv comprising SEQ ID NO:2 (VH) and SEQ ID NO:3 (VL).

In a further aspect the present invention provides a nucleic acid encoding an antibody or antigen binding fragment thereof specific for the amino acid sequence set forth in SEQ ID NO:1 (the Whitlow linker sequence), wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.

Said nucleic acid, wherein said antibody or antigen binding fragment thereof is a scFv comprising SEQ ID NO:2 (VH) and SEQ ID NO:3 (VL).

In a further aspect the present invention provides an in vitro method for detection, analysis and or separation (isolation) of immune cells in a sample, wherein said immune cells express a chimeric antigen receptor (CAR) comprising an extracellular antigen binding domain comprising the amino acid sequence set forth in SEQ ID NO:1 (Whitlow linker), the method comprising
i) contacting the CAR expressing immune cells of said sample with an antibody or antigen binding fragment thereof specific for the amino acid sequence set forth in SEQ ID NO:1 (the Whitlow linker sequence), wherein said antibody or antigen binding fragment thereof comprises
   a) a heavy chain variable region of an antibody (VH) comprising SEQ ID NO:2, and
   b) a light chain variable region of an antibody (VL) comprising SEQ ID NO:3,
ii) determining, analyzing and/or separating (isolating) the CAR expressing immune cells of said sample.

Said method, wherein said antigen binding fragment of said antibody is a antibody is a Fab (fragment antigen binding), scFv (single chain fragment variable), Fab' or F(ab')2.

Said method, wherein said antibody or antigen binding fragment thereof is a scFv comprising SEQ ID NO:2 (VH) and SEQ ID NO:3 (VL).

Said method, wherein said antibody or antigen binding fragment thereof is a scFv comprising SEQ ID NO:2 (VH) and SEQ ID NO:3 (VL) in the order from N-terminus to C-terminus SEQ ID NO:2 (VH) and SEQ ID NO:3 (VL).

Said method, wherein said antibody or antigen binding fragment thereof is a scFv comprising SEQ ID NO:2 (VH) and SEQ ID NO:3 (VL) in the order from N-terminus to C-terminus SEQ ID NO:3 (VL) and SEQ ID NO:2 (VH).

Said method, wherein said antibody or antigen binding fragment thereof is coupled to a detection moiety.

Said method, wherein antibody or antigen binding fragment thereof is coupled to a solid phase.

Said method, wherein said detection moiety is a chromophore, a fluorescent moiety, a phosphorescent moiety, a luminescent moiety, a light absorbing moiety, a radioactive moiety, a transition metal isotope mass tag moiety, or a magnetic particle.

Said method, wherein said immune cells are T cell, NK cells or HSCs.

Said method, wherein said immune cells of said sample are thawed immune cells that have been frozen beforehand after a process of generation of CAR immune cells.

Said method, wherein said immune cells of said sample are thawed immune cells, that are cultured (e.g. for 2 to 14 days) before said CAR expressing immune cells are contacted with said antibody or antigen binding fragment thereof.

Said method, wherein said immune cells of said sample are fresh immune cells.

In another aspect the present invention provides the use of an antibody or antigen binding fragment thereof for detection, analysis and/or separation of immune cells in a sample, wherein said immune cells express a chimeric antigen receptor (CAR) comprising an extracellular antigen binding domain comprising the amino acid sequence set forth in SEQ ID NO:1 (Whitlow linker), wherein said antibody or antigen binding fragment thereof is specific for the amino acid sequence set forth in SEQ ID NO:1 (the Whitlow linker sequence), wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.

Said use, wherein said antibody or antigen binding fragment thereof is a scFv comprising SEQ ID NO:2 (VH) and SEQ ID NO:3 (VL).

### Generation of of CAR immune cells such as CAR T cells

Processes of generation of CAR immune cells such as CAR T cells are well known in the art. Exemplarily in the following methods of generation of immune cells expressing a CAR are disclosed.

The genetically modified immune cells expressing the CAR as disclosed herein, preferentially T cells, may be generated preferentially in an automated process in a closed system. A process for the generation of genetically modified cells, preferentially T cells, is disclosed e.g. in WO2015162211A1 and may comprise the e.g. steps:
a) providing a cell sample comprising immune cells (e.g. from a PBMC)
b) preparation of the cell sample by centrifugation
c) magnetic separation of the immune cells, preferentially T cells,
d) activation of the enriched immune cells, preferentially T cells, using modulatory agents
e) genetically modifying the immune cells, preferentially T cells, to express the CAR as disclosed herein
f) expansion of the genetically modified immune cells, preferentially T cells, in a cultivation chamber
g) washing of the cultured immune cells, preferentially T cells.

All these steps may be performed automatically in a closed system.

The process is especially suited for preparing gene modified cells such as immune cells, preferentially T cells, wherein the enriched immune cells, preferentially T cells, are gene-modified by using viral and/or non-viral vectors, preferentially using a lentiviral vector.

In case of magnetically enrichment of T cells from PBMC or leukapheresis anti-CD4 and/or anti-CD8 antibodies or antigen binding fragments coupled to beads may be used.

The modulatory agents may be selected from agonistic antibodies such as anti-CD3 and/or anti-CD28 antibodies or antigen binding fragments thereof (especially in case of modifying T cells), and/or cytokines.

The gene-modified immune cells, preferentially T cells, may be enriched by magnetic labelling of immune cells and magnetic separation before or after cultivation to obtain higher frequency of gene-modified immune cells, preferentially T cells, in the final cellular product.

The cultivation (expansion) may be over several day such as 8 to 12 days, or may be a shorter cultivation process without or with less cultivation/expansion as disclosed e.g. in WO2020239866A1. In case of a shorter in-vitro process of generation of immune cells such as T cells, the generated immune cells such as T cells may expand *in-vivo* after administration to a subject in need thereof to therapeutically effect amounts of immune cells expressing the CAR as disclosed herein (see e.g. WO2020239866A1) . Such a short *ex-vivo* process may comprise e.g. (in a closed system for cell modification) the steps
a) providing a sample (e.g. from PBMC) comprising immune cells such as T cells
b) preparation of said sample by centrifugation
c) enrichment of the immune cells such as T cells of step b
d) activation of the enriched immune cells such as T cells using modulatory agents
e) genetic modification of the activated immune cells such as T cells by transduction e.g. with lentiviral vector particles
f) removal of said modulatory agents,
thereby generating a sample of genetically modified immune cells such as T cells, wherein said method is performed e.g. in equal or less than 3 days (72h).

As a closed system for cell modification, the fully automated cell processing device CliniMACS Prodigy^{®} and associated tubing sets (Miltenyi Biotec B.V. & Co. KG, Germany) may be used (WO2009/072003). This closed system meets the requirements of GMP-grade processing of almost any kind of cellular products and may allow reducing clean room requirements, improve technology transfer and harmonization of cell manufacturing processes.

### Chimeric antigen receptor with the Whitlow 218 linker sequence

In general, a CAR may comprise an extracellular domain (extracellular part) comprising the antigen binding domain, a transmembrane domain and a cytoplasmic signaling domain (intracellular signaling domain). The extracellular domain may be linked to the transmembrane domain by a spacer (or hinge region). The extracellular domain may also comprise a signal peptide.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA). The CARs may comprise one or more antigen binding domains (e.g. a tandem CAR). Generally, the targeting regions on the CAR are extracellular. The antigen binding domain may comprise an antibody or an antigen binding fragment thereof. Generally, the antigen binding domain of a CSAR may comprise, for example, full length heavy chain, Fab fragments, single chain Fv (scFv) fragments, divalent single chain antibodies, nanobodies or diabodies. Often the antigen binding domain is a scFv. Normally, in a scFv the variable regions of an immunoglobulin heavy chain and light chain are fused by a flexible linker to form a scFv. Herein, the CARs require an antigen binding domain comprising a scFv comprising a VH and VL fused via the "Whitlow 218 linker", so that the antibody or antigen binding fragment thereof as disclosed herein can bind to the CAR via the Whitlow 218 linker.

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain and the transmembrane domain. The CARs may comprise an extracellular spacer domain but is it also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The splitting key signaling and antigen recognition modules enable for a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are that those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some embodiments, the endodomain may contain a primary cytoplasmic signaling domains or a co-stimulatory region, but not both.

The CARs of the present disclosures may be designed to comprise any portion or part of the above-mentioned domains as described herein in any order and/or combination resulting in a functional CAR, i.e. a CAR that mediated an immune effector response of the immune effector cell that expresses the CAR as disclosed herein.

The engineered cell expressing a CAR may be further modified by genetic engineering using methods well known in the art e.g. Meganucleases, TALEN, CrisprCas, zink finger nucleases, shRNA and /or miRNA. Said cells may be modified to reduce or lack expression of a specific gene, which is normally expressed in the cell e.g. T cell receptor (TCR), MHC, co-inhibitory molecules like PD-1, CTLA-4, BTLA, TIGIT, Tim-3, CD244, LAIR, Lag-3, CD160, HVEM .

Said cells may be modified to express additional transgenes such as therapeutic controls, cytokines and/or fragments, cytokine receptors and/or fragments, cytokine receptor fusion proteins, costimulatory receptors or armoring molecules.

### Methods/assays using the antibody or an antigen binding fragment thereof specific for the Whitlow linker of a CAR expressed in an immune cell

The antibody or an antigen binding fragment thereof specific for the Whitlow linker of a CAR expressed in an immune cell as disclosed herein may be used in many different methods and/or assays that aim for e.g. in-process control (IPC), quality control (QC), immune-monitoring, concomitant research and further analytics of the CAR immune cells.

Exemplary some methods/assays are described herein in more detail without intention to be limited to these methods/assays.

### Magnetic cell separation of CAR immune cells

CAR immune cells such as CAR T cells may be generated by methods known in the art, e.g. as described herein.

These CAR immune cells comprise the Whitlow linker in the extracellular antigen binding domain of the CAR. This allows for the use of the antibody or an antigen binding fragment thereof specific for SEQ ID NO:1 as disclosed herein for enrichment of the immune cells expressing the CAR.

The sample comprising CAR expressing immune cells and immune cells that do not express the CAR are contacted with said antibody or an antigen binding fragment thereof specific for SEQ ID NO: 1, wherein said antibody or an antigen binding fragment thereof is coupled to a magnetic bead, e.g. with colloidal superparamagnetic microparticles of the Magnetic-activated cell sorting (MACS) technology (Miltenyi Biotec B.V. & Co. KG, Germany).

The MACS technology allows cells to be separated by incubating them with magnetic nanoparticles coated with antibodies directed against a particular surface antigen, here the amino acid sequence of the Whitlow linker. This causes the immune cells expressing this antigen, i.e. that express the CAR, to attach to the magnetic nanoparticles. Afterwards the cell solution is transferred on a column placed in a strong magnetic field. In this step, the immune cells expressing the CAR attach to the nanoparticles and stay on the column, while other cells (not expressing the antigen) flow through. With this method, the cells can be separated positively or negatively with respect to the particular antigen(s)/marker(s).

In case of a positive selection the immune cells expressing the CAR, which are attached to the magnetic column, are washed out to a separate vessel, after removing the column from the magnetic field, thereby (further) enriching the immune Cells expressing the CAR.

### In-process control (IPC) and quality control (QC)

Manufacturing of CAR T cells is a tightly regulated process. To minimize the risk of manufacturing failure, in-process controls (IPCs) are required at certain timepoints throughout CAR T cell production. One particularly relevant parameter is the transduction frequency, describing which fraction of T cells (viable CD3+ & CD45+) is expressing the engineered CAR.

The transduction frequency can be assessed by flow cytometry using linker-specific antibodies as described herein and typically has to fall into an pre-defined acceptance range.

Likewise, for the final drug formulation a quality control requires again the assessment of the CAR transduction frequency. Based on CAR transduction frequency and concentration of viable T cells, the individual dose of CAR T cells can be formulated for the patient.

### Immune-monitoring

Immune-monitoring after adoptive cell transfer, such as CAR T cell therapy, involves tracking the behavior and persistence of the infused CAR T cells in patients to assess treatment efficacy and safety. This is typically done using methods like flow cytometry and real-time PCR to measure CAR T cell levels and their functional activity over time in the blood. Immune-monitoring helps guide clinical decisions by evaluating CAR T cell expansion, persistence, and the immune system's dynamic reconstruction after therapy. In order to detect and enumerate the frequency of persistent CAR T cells in patient blood samples via flow cytometry, CAR Detection Reagents are used. These CAR Detection Reagents need to be specific for the used CAR and can also target common linker sequences as the Whitlow-linker.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

As used herein, the term "about" will be understood by persons of ordinary skill in the art and will vary to some extent on the context in which it is used. As used herein, "about" when referring to a measurable value such as an amount, a temporal duration, and the like, is meant to encompass variations of ±20% or ±10%, more preferably ±5%, even more preferably ±1% from the specified value

The terms "Whitlow 218 linker" or Whitlow linker" or "Whitlow" may be used herein interchangeably.

The Whitlow 218 linker is a commonly used linker (peptide) that connects the variable heavy (VH) domain and variable light (VL) domain of single-chain variable fragments (scFvs) and chimeric antigen receptors (CARs) that utilize an extracellular domain scFv for target antigen recognition. The Whitlow 218 linker affords the scFv with enhanced resistance to proteolysis and aggregation. The amino acid sequence of the Whitlow linker is set forth in SEQ ID NO:1. The Whitlow linker may also be present in other fusion proteins, e.g. connecting two different domains of a protein or of proteins that are not connected naturally.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (VHH and nanobodies), diabodies, dsFv, Fab', F(ab')2, single-chain antibody molecules, and multispecific antibodies formed from antibody fragments.

A "humanized" antibody or antigen binding fragment includes a human framework region and one or more CDRs from a non-human (such as a mouse, rat, or synthetic) antibody or antigen binding fragment. The non-human antibody or antigen binding fragment providing the CDRs is termed a "donor," and the human antibody or antigen binding fragment providing the framework is termed an "acceptor." In one embodiment, all the CDRs are from the donor immunoglobulin in a humanized immunoglobulin. Constant regions need not be present, but if they are, they can be substantially identical to human immunoglobulin constant regions, such as at least about 85-90%, such as about 95% or more identical. Hence, all parts of a humanized antibody or antigen binding fragment, except possibly the CDRs, are substantially identical to corresponding parts of natural human antibody sequences.

A "fully human antibody" or "human antibody" is an antibody or antigen binding fragment thereof which includes sequences from (or derived from) the human genome, and does not include sequence from another species. In some embodiments, a human antibody includes CDRs, framework regions, and (if present) an Fc region from (or derived from) the human genome. Human antibodies can be identified and isolated using technologies for creating antibodies based on sequences derived from the human genome, for example by phage display or using transgenic animals.

The term "CDR" denotes a complementarity determining region as defined by at least one manner of identification to one of skill in the art. The precise amino acid sequence boundaries of a given CDR or framework region (FR) can be readily determined using any of a number of well-known schemes, including the numbering system of Kabat.

As used herein, the term "antigen" is intended to include substances that bind to or evoke the production of one or more antibodies and may comprise, but is not limited to, proteins, peptides, polypeptides, oligopeptides, lipids, carbohydrates such as dextran, and combinations thereof, for example a glycosylated protein or a glycolipid. The term "antigen" as used herein refers to a molecular entity that may be expressed on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "expression" as used herein is defined as the transcription and/or translation of a particular nucleotide sequence driven by its promoter in a cell.

As used herein, the term "subject" refers to an animal. Preferentially, the subject is a mammal such as mouse, rat, cow, pig, goat, chicken, dog, monkey or human. More preferentially, the individual is a human. The subject may be a subject suffering from a disease such as cancer.

The terms "nucleic acid", "nucleic acid sequence", "nucleic acid molecule" or "polynucleotide" may be used interchangeably herein and refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means.

A recombinant nucleic acid may be one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, e.g., by genetic engineering techniques well-known in the art.

A recombinant protein is a biotechnologically generated protein that does not occur naturally in a eukaryotic and/or prokaryotic cell. Often it is composed of different domains from different proteins, e.g. as used herein, a viral envelope protein is fused (at its ectodomain) to a polypeptide that comprises an antigen binding domain specific for an antigen.

The term "transduction" means the transfer of genetic material from a viral agent such as a lentiviral vector particle into a eukaryotic cell such as a T cell.

The terms "having specificity for", "specifically binds" or "specific for" with respect to an antigen-binding domain of an antibody or a fragment thereof refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain as specific. An antigen-binding domain that specifically binds to an antigen may bind also to different allelic forms of the antigen (allelic variants, splice variants, isoforms etc.). This cross reactivity is not contrary to the definition of that antigen-binding domain as specific.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based cytotoxic responses to attack cancer cells. T cells that have a natural or genetically engineered reactivity to a patient's cancer are generated in vitro and then transferred back into the cancer patient or are directly generated in-vivo. Then the immunotherapy is referred to as "CAR T cell immunotherapy".

The term "treatment" as used herein means to reduce the frequency or severity of at least one sign or symptom of a disease.

The term "(therapeutically) effective amount" as used herein means an amount of a pharmaceutical composition which is sufficient to significantly and positively modify the symptoms and/or conditions to be treated. The effective amount of an active ingredient such a genetically modified immune cell for use in a pharmaceutical composition will vary with the particular condition being treated, the severity of the condition, the duration of treatment, the nature of concurrent therapy, the particular active ingredient(s) being employed, the particular pharmaceutically-acceptable carrier(s) utilized.

The terms "engineered cell" and "(genetically) modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface.

The terms "immune cell" or "immune effector cell" may be used interchangeably and refer to a cell that may be part of the immune system and executes a particular effector function such as T cells, alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and/or NK cells. Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs are genetically engineered as disclosed herein, and then given back to the patient. "Effector function" means a specialized function of a cell, e.g. in a T cell an effector function may be cytolytic activity or helper activity including the secretion of cytokines.

T cells or T lymphocytes are a type of lymphocyte that play a central role in cell-mediated immunity. They can be distinguished from other lymphocytes, such as B cells and natural killer cells (NK cells), by the presence of a T cell receptor (TCR) on the cell surface. There are several subsets of T cells, each with a distinct function.

T helper cells (TH cells) assist other white blood cells in immunologic processes, including maturation of B cells into plasma cells and memory B cells, and activation of cytotoxic T cells and macrophages. These cells are also known as CD4+ T cells because they express the CD4 glycoprotein on their surface. Helper T cells become activated when they are presented with peptide antigens by MHC class II molecules, which are expressed on the surface of antigen-presenting cells (APCs). Once activated, they divide rapidly and secrete small proteins called cytokines that regulate or assist in the active immune response. These cells can differentiate into one of several subtypes, including TH1, TH2, TH3, TH17, Th9, or TFH, which secrete different cytokines to facilitate a different type of immune response. Signaling from the APC directs T cells into particular subtypes.

Cytotoxic T cells (TC cells, or CTLs) destroy virally infected cells and tumor cells and are also implicated in transplant rejection. These cells are also known as CD8+ T cells since they express the CD8 glycoprotein at their surface. These cells recognize their targets by binding to antigen associated with MHC class I molecules, which are present on the surface of all nucleated cells.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - Foxp3+ Treg cells and Foxp3-Treg cells.

Natural killer T cells (NKT cells - not to be confused with natural killer cells of the innate immune system) bridge the adaptive immune system with the innate immune system. Unlike conventional T cells that recognize peptide antigens presented by major histocompatibility complex (MHC) molecules, NKT cells recognize glycolipid antigen presented by a molecule called CD1d. Once activated, these cells can perform functions ascribed to both Th and Tc cells (i.e., cytokine production and release of cytolytic/cell killing molecules).

Natural killer cells (NK cells) are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter the circulation. NK cells differ from natural killer T cells (NKTs) phenotypically, by origin and by respective effector functions; often, NKT cell activity promotes NK cell activity by secreting IFNy. In contrast to NKT cells, NK cells do not express T cell antigen receptors (TCR) or pan T marker CD3 or surface immunoglobulins (Ig) B cell receptors, but they usually express the surface markers CD16 (FcyRIII) and CD56 in humans, NK1.1 or NK1.2 in C57BL/6 mice. Up to 80% of human NK cells also express CD8. Continuously growing NK cell lines can be established from cancer patients and common NK cell lines are for instance NK-92, NKL and YTS.

The term "isolated" is used herein to indicate that the polypeptide, nucleic acid or host cell exist in a physical milieu distinct from that in which it occurs in nature. For example, the isolated polypeptide may be substantially isolated (for example enriched or purified) with respect to the complex cellular milieu in which it naturally occurs, such as in a crude extract.

A transgene may be a gene that has been transferred by genetic engineering techniques into a host cell that normally does not bear this gene. The gene may be a naturally gene that occurs in other cells or may be a recombinant gene. Normally the transgenes used in the present invention may be the chimeric antigen receptor comprising the Whitlow 218 linker as disclosed herein. The expressed transgene may also be referred to a heterologous protein or transgenic polypeptide.

The term "sample" as used herein may refer to a cell sample comprising immune cells that express a chimeric antigen receptor (CAR), wherein said CAR comprises an extracellular antigen binding domain comprising the amino acid sequence set forth in SEQ ID NO:1 (Whitlow linker).Said sample may comprise also other cells, e.g. immune cells that does not comprise the expression of a CAR.

The sample may be a fresh sample directly analyzed in a method as disclosed herein after the generation of immune cells that express said CAR, or that sample may be a sample that has been frozen after generation of immune cells expressing said CAR and subsequently thawed before use in a method as disclosed herein. Alternatively, the sample may be e.g. a blood sample taken from a patient that has been treated with a CAR immune cell therapy for immune-monitoring of the CAR immune cells of said patient.

The term "solid phase" as used herein refers to the coupling of the antigen-binding fragment, e.g. an antibody, to other molecules, e.g. particles, fluorophores, haptens like biotin, or larger surfaces such as culture dishes and microtiterplates. In some cases the coupling results in direct immobilization of the antigen-binding fragment, e.g. if the antigen-binding fragment is coupled to a larger surface of a culture dish. In other cases this coupling results in indirect immobilisation, e.g. an antigen-binding fragment coupled directly or indirectly (via e.g. biotin) to a magnetic bead is immobilised if said bead is retained in a magnetic field. In further cases the coupling of the antigen-binding fragment to other molecules results not in a direct or indirect immobilization but allows for enrichment, separation, isolation, and detection of cells according to the present invention, e.g. if the antigen-binding fragment is coupled to a fluorophore which then allows discrimination of labelled cells and non-labelled cells, e.g. via flow cytometry methods, like FACSsorting, or fluorescence microscopy.

The term "particle" as used herein refers to a solid phase such as colloidal particles, microspheres, nanoparticles, or beads. Methods for generation of such particles are well known in the field of the art. The particles may be magnetic particles. The particles may be in a solution or suspension or they may be in a lyophilised state prior to use in the present invention. The lyophilized particle is then reconstituted in convenient buffer before contacting the sample to be processed regarding the present invention.

The term "magnetic" in "magnetic particle" as used herein refers to all subtypes of magnetic particles which can be prepared with methods well known to the skilled person in the art, especially ferromagnetic particles, superparamagnetic particles and paramagnetic particles. "Ferromagnetic" materials are strongly susceptible to magnetic fields and are capable of retaining magnetic properties when the field is removed. "Paramagnetic" materials have only a weak magnetic susceptibility and when the field is removed quickly lose their weak magnetism. "Superparamagnetic" materials are highly magnetically susceptible, i.e. they become strongly magnetic when placed in a magnetic field, but, like paramagnetic materials, rapidly lose their magnetism.

The linkage between antibody and particle can be covalent or non-covalent. A covalent linkage can be, e.g. the linkage to carboxyl-groups on polystyrene beads, or to NH₂ or SH₂ groups on modified beads. A non-covalent linkage is e.g. via biotin-avidin or a fluorophore-coupled-particle linked to anti-fluorophore antibody. Methods for coupling antibodies to particles, fluorophores, haptens like biotin or larger surfaces such as culture dishes are well known to the skilled person in the art.

For enrichment, isolation or selection in principle any sorting technology can be used. This includes for example affinity chromatography or any other antibody-dependent separation technique known in the art. Any ligand-dependent separation technique known in the art may be used in conjunction with both positive and negative separation techniques that rely on the physical properties of the cells. An especially potent sorting technology is magnetic cell sorting. Methods to separate cells magnetically are commercially available e.g. from Invitrogen, Stem cell Technologies, in Cellpro, Seattle or Advanced Magnetics, Boston. For example, monoclonal antibodies can be directly coupled to magnetic polystyrene particles like Dynal M 450 or similar magnetic particles and used e.g. for cell separation. The Dynabeads technology is not column based, instead these magnetic beads with attached cells enjoy liquid phase kinetics in a sample tube, and the cells are isolated by placing the tube on a magnetic rack. However, in a preferred embodiment for enriching, sorting and/or detecting target cells from a sample containing target cells antibodies or antigen binding fragments thereof are used in conjunction with colloidal superparamagnetic microparticles having an organic coating by e.g. polysaccharides (Magnetic-activated cell sorting (MACS) technology (Miltenyi Biotec, Bergisch Gladbach, Germany)). These particles (nanobeads or MicroBeads) can be either directly conjugated to monoclonal antibodies or used in combination with anti-immunoglobulin, avidin or anti-hapten-specific MicroBeads.

The MACS technology allows cells to be separated by incubating them with magnetic nanoparticles coated with antibodies directed against a particular surface antigen. This causes the cells expressing this antigen to attach to the magnetic nanoparticles. Afterwards the cell solution is transferred on a column placed in a strong magnetic field. In this step, the cells attach to the nanoparticles (expressing the antigen) and stay on the column, while other cells (not expressing the antigen) flow through. With this method, the cells can be separated positively or negatively with respect to the particular antigen(s).

In case of a positive selection the cells expressing the antigen(s) of interest, which attached to the magnetic column, are washed out to a separate vessel, after removing the column from the magnetic field.

In case of a negative selection the antibody used is directed against surface antigen(s) which are known to be present on cells that are not of interest. After application of the cells/magnetic nanoparticles solution onto the column the cells expressing these antigens bind to the column and the fraction that goes through is collected, as it contains the cells of interest. As these cells are non-labelled by an antibody coupled to nanoparticles, they are "untouched".

The procedure can be performed using direct magnetic labelling or indirect magnetic labelling. For direct labelling the specific antibody is directly coupled to the magnetic particle. Indirect labelling is a convenient alternative when direct magnetic labelling is not possible or not desired. A primary antibody, a specific monoclonal or polyclonal antibody, a combination of primary antibodies, directed against any cell surface marker can be used for this labelling strategy. The primary antibody can either be unconjugated, biotinylated, or fluorophore-conjugated. The magnetic labelling is then achieved with anti-immunoglobulin MicroBeads, anti-biotin MicroBeads, or anti-fluorophore MicroBeads.

The terms "detection moiety" refers to any moiety possessing a property or function which can be used for detection purposes. Said detection moiety may be attached, by a covalent or non-covalent coupling, directly or indirectly to another molecule, preferentially to an antibody or antigen binding fragment thereof. Said detection moiety may be used, for example, to detect, trace, separate, isolate, deplete, analyze, or quantify the labeled molecule and thereby the antigen-comprising biological specimen. For example stronger labeled cells, weaker labeled cells, and non-labeled cells may be discriminated and/or isolated, e.g. via flow cytometry, fluorescent cell sorting, magnetic cell sorting, or fluorescence microscopy.

The detection moiety may be selected from a group consisting of chromophore, fluorescent moiety, phosphorescent moiety, luminescent moiety, light absorbing moiety, radioactive moiety, transition metal isotope mass tag moiety, and magnetic particle. Preferably, said detection moiety may be a chromophore, fluorescent moiety, phosphorescent moiety, luminescent moiety, light absorbing moiety, or particle. More preferred, said detection moiety may be a fluorescent moiety, phosphorescent moiety, luminescent moiety, light absorbing moiety, or magnetic particle. Most preferred, said detection moiety may be a fluorescent moiety, luminescent moiety, light absorbing moiety, or magnetic particle. Detection moieties may be combined, for example a magnetic particle may be used in combination with a fluorescent moiety. In this case, it is sufficient when the antibody or antigen binding fragment thereof is coupled to at least one of the combined detection moieties.

Useful fluorescent moieties might be protein-based, such as phycobiliproteins, polymeric, such as polyfluorenes, small organic molecule dyes, such as xanthenes, like fluorescein, or rhodamines, cyanines, oxazines, coumarins, acridines, oxadiazoles, pyrenes, pyrromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties. Another group of photoluminescent detection moieties are phosphorescent moieties with time-delayed emission of light after excitation. Phosphorescent moieties include metallo-organic complexes, such as Pd, Pt, Tb, Eu complexes, or nanoparticles with incorporated phosphorescent pigments such as lanthanide doped SrAl₂O₄. Radioactive moieties may be in the form of radioisotope labeling by exchanging nonradioactive isotopes for their radioactive counterparts, such as tritium, 32P, 35S or 14C, or introducing covalently bound labels, such as 125I, which is bound to tyrosine, 18F within fluorodeoxyglucose, or metallo-organic complexes, i.e. 99Tc-DTPA. A detection moiety may be capable of causing chemiluminescence, i.e. horseradish peroxidase label in the presence of luminol, or may be capable of absorbing of UV, visible light, or NIR radiation. Suitable light-absorbing detection moieties are light absorbing dyes without fluorescence emission, such as small organic molecule quencher dyes like N-aryl rhodamines, azo dyes, and stilbenes. A light-absorbing detection moiety may be irradiated by pulsed laser light, generating an photoacoustic signal. An isotope mass tag moiety may be detected by mass spectrometric detection of a transition metal isotope. Transition metal isotope mass tag labels might be introduced as covalently bound metallo-organic complexes or nanoparticle component. Known in the art are isotope tags of lanthanides and adjacent late transition elements.

Fluorescent moieties bound to the antigen during the method as disclosed herein may be detected, e.g., under a fluorescence microscope, in a flow cytometer, a spectrofluorometer, or a fluorescence scanner.

A "kit" may comprise a container with components within the container. Such containers may be e.g. boxes, bottles, vials, tubes, bags, pouches, blister packs, or other suitable container forms known in the art. Such containers may be made of plastic, glass, laminated paper, metal foil, or other materials suitable for holding components therein. The kit further may comprise written directions for using the components of the kits.

### Examples

The following examples are intended for a more detailed explanation of the invention but without restricting the invention to these examples.

### Example 1: Identification of antibody fragments

The anti-Whitlow-Linker candidate selection process consisted of an initial phage display selection, followed by a flow cytometric screening, to ensure specificity of the candidates to CAR-Whitlow(+) transduced Sup-T1 cells compared to CAR-Whitlow(-) SupT1 wt cells. Following, further in vitro analysis with fluorochrome coupled candidates allowed to identify two novel, functional and specific anti-Whitlow-Linker candidates (Fig 1).

Two human naïve scFv phage display libraries constructed at Miltenyi Biotec were used for the identification of novel binders against Whitlow-Linker. These libraries differed in the variable domains of the light chain, they contained either the κ or the λ isotype. The library was constructed from naïve human B-cells of more than 120 healthy donors, resulting in an antibody diversity of 5 x 10¹⁰. For panning, two biotinylated peptides (Whitlow_1 and Whitlow_2) were synthesized at Miltenyi Biotec. Additionally one scFv (containing Whitlow-Linker) produced at Miltenyi Biotec was used. Streptavidin (VWR Chemicals; Cat. no. E497) was purchased from VWR/Avantor. Three different panning strategies were used to identify binder candidates using Whitlow-Linker variants for positive selection. In all strategies Streptavidin antigen was used for competition (Fig. 2A). For panning, each library was incubated on captured antigens using Streptavidin immobilized on MaxiSorb ELISA Plates (Nunc). After washing, scFv phage particles were eluted using trypsin. Eluted phage particles were used to infect *E. coli* TG1 and phage production was facilitated using M13K07 helper phage. After the third panning round eluted phage particles were used to infect *E. coli* TG1 and single cell colonies were inoculated the next day for expression of scFvs.

Expressed scFvs were analyzed for their capability to bind Whitlow_1 (Miltenyi Biotec), Whitlow_2 (Miltenyi Biotec), Whitlow_scFv (Miltenyi Biotec) or Streptavidin in ELISA. This lead to the identification of 322 first hits that bind Whitlow-Linker and do not bind Streptavidin. Separating ELISA hits by antigen variant revealed, that binders selected from all strategies do show binding to both Whitlow-Linker variants, the peptide variant Whitlow_1 (Miltenyi Biotec) as well as the full protein variant Whitlow_scFv (Miltenyi Biotec) in ELISA (Fig. 2B). 322 first hit scFv genes were sequenced resulting in 305 processable sequences. Alignment of the scFv genes revealed that 55 out of 305 clones contain unique scFv sequences (Fig. 2C). Identified unique candidates were cloned as scFv-Fc fusion into mammalian expression vectors. After expression and purification, 34 of these scFv-Fc proteins were used for analysis by flow cytometry.

### Example 2: Expression and purification of anti-Whitlow scFv-Fc candidates

Potential anti-Whitlow scFv-Fc candidates were manufactured via transient transfection of HEK293 cells. On the day of transfection, HEK293 cells were seeded at a density of 2x10⁶ cells per well in a total volume of 800 µl expression medium in a 96 deep well plate. Next, transfection reagent was mixed with diluted plasmid DNA, encoding for the respective anti-Whitlow scFv-Fc candidate and added to the cells after an incubation period of 10 minutes. Cells were incubated at 37°C and 8% CO2 on an orbital shaker. Supernatants were collected on day 4 after transfection and scFv-Fc candidates were purified using Protein A PhyTip^{®} columns (Biotage) in combination with an automated workstation (Beckman Coulter). Purity was confirmed via SDS page and protein concentrations were determined using a microplate reader by measuring UV absorbance at 280 nm. Purified proteins were stored at -20°C until further processing.

### Example 3: Flow cytometric screening of anti-Whitlow scFv-Fc candidates

Anti-Whitlow-Linker scFv-Fc candidates were manufactured as described in example 2 and adjusted to an equal protein concentration. For flow cytometric validation, non-target cells were labeled with CellTrace^{™} Violet or Cell Trace^{™} FarRed (ThermoFisher) and 1x10⁵ cells per cell line were seeded into a 96-well plate. Cells were pelleted at 1200 g for 2 min at 4°C and stained with 50 µL staining mixture containing 100 nM scFv-Fc. Staining was performed for 30 min at 4°C followed by two successive washing steps with 150 µL PBS / 2 mM EDTA / 0.5% BSA (referred to as PEB, 1200 g, 2 min). Next, 50 µl of secondary staining mixture comprising the AffiniPure F(ab')₂ Fragment Goat Anti-Human IgG-APC, Fcy fragment specific (Jackson ImmunoResearch; Polyclonal, Cat. no. 109-136-098) as well as 7-AAD as dead cell marker (Miltenyi Biotec) was added to the cells. Staining was performed for 10 min at 4°C followed by two successive washing steps with 150 µL PEB. Cells were resuspended in 50 µL PEB for subsequent flow cytometric analysis at the MACSQuant^{®} Analyzer 10 (Miltenyi Biotec).

Cells were gated on size, singularity and viability before distinguishing between the CAR-Whitlow(+) transduced Sup-T1 cell line and the Whitlow(-) SupT1 wt cell line via CellTrace. Staining of the respective Whitlow-Linker positive target cell line with the anti-Whitlow-Linker scFv-Fc candidates was displayed as MFI and frequency (%) of stained cells. All 34 anti-Whitlow scFv-Fc candidates, which had been successfully produced and purified, showed a positive result (MFI > 10; frequency > 70%) on CAR-Whitlow(+) transduced Sup-T1 cells in flow cytometric analysis (Fig. 3A). None of the candidates showed unspecific staining (MFI > 10; frequency > 5%) on Whitlow(-) SupT1 wt cells (Fig. 3B). Aiming for anti-Whitlow-Linker scFv-Fc candidates that show specific binding for human CAR-Whitlow(+) transduced Sup-T1 cells, but no signal on Whitlow(-) SupT1 wt cells, in total all 34 candidates (black bars) were selected for further analysis.

### Example 4: Generation and identification of one lead anti-Whitlow-Linker full IgG antibodies for Whitlow-Linker expressing target cell recognition.

### PE conjugation of anti Whitlow Binder:

Conjugation was done according to standard protocols for antibody-PE conjugation. First, the reduction of antibody with was done (e.g. DTT). For the successful reduction of antibody, the antibody had to be adjusted to a concentration of 4 mg/ mL. After the reduction process, the antibody was directly conjugated to PE. As an in-process control, the average number of SH-groups on the antibody was determined. The SH-groups were calculated to be in a range of 5-10 thiols per molecule.

In parallel, the activation of PE with SMCC was done. PE was provided as (NH₄)₂SO₄ precipitate and was centrifuged before use, dialyzed and concentration and purity of the PE was determined by OD565 via UV/Vis spectroscopy. The concentration of PE was adjusted to 6 mg/ mL and the activation of PE by SMCC was conducted at a molecular ratio of ~5:1. The calculated volume of SMCC solution was added to the PE solution, mixed and incubated at RT in the dark on a shaker for 1 hour. Subsequently, rebuffering and removal of free SMCC from the PE was done by purification with a PBS/EDTA-equilibrated PD10 Sephadex column. The concentration of the activated PE was determined by measuring OD565 via UV/Vis spectroscopy. The quantification of maleimide groups was done by using activated and non-activated PE which was incubated with Cysteamine chloride solution The average number of maleimide-groups per PE molecule was calculated to be ~1-2 as determined by UV/Vis spectroscopy (OD412). The conjugation was conducted at an antibody concentration of 0.4 mg/mL with a molecular coupling rate of 1:2. for 1 hour at RT in the dark on a shaker.

The reaction batch was stopped by adding β-Mercaptoethanol and N-Ethylmaleimide (NEM) solution.

Size exclusion chromatography was performed using a Superdex 200 column to remove unreacted PE, unreacted antibody or aggregates. The fractions were pooled after the chromatogram in a size range corresponding to ~670-300 kDa. Aggregates, free PE and free antibody were excluded. The best-performing fractions were finally pooled and re-tested on cells to define the filling concentration.

### Cell Staining of PE conjugated Whitlow Binder PE:

To assess the performance of the different PE-conjugated anti Whitlow Binders (clone MB524-B07, clone MB524-A08 and a commercial clone E3U7Q), titrations were conducted on target-expressing cell lines (0.05 - 10 µg/mL). For this purpose, SupT1 cells that stably express a CAR containing a Whitlow-linker within the scFv were stained with the conjugates along with non-transduced wt-SupT1 cells. 1x10E6 cells were stained with the appropriate volume of staining mixture (CD4-APCVio770/ CD8-VioGreen/ 7-AAD) and anti Whitlow Binder-PE conjugate. CAR-Whitlow transduced SupT1 cells and SupT1 wildtype cells were stained separately. For exclusion of dead cells, 7-AAD was used. After an incubation at RT in the dark for 10 min, the samples were washed. Subsequently, the cells were resuspended and cells were directly analyzed by flow cytometry using the MACSQuant^{®} Analyzer10. CAR-transduced SupT1 cells and wt SupT1 cells were mixed 1:1 directly before flow cytometric analysis.

A concentration-dependent binding curve was observed for all tested conjugates. The staining index was used as a readout for staining performance. Clone MB524-B07 and clone MB524-A08 reached saturated binding at ~2µg/ml, whereas saturated binding was not reached with the commercially available clone E702V at the tested concentration range. The staining index of MB524-B07 was superior over MB524-A08 and also E3U7Q at the manufacturer's recommended titer (see Fig 7).

Similarly, the staining index of MB524-B07 was superior over MB524-A08 and the clone 16 of US20200048681 (see Fig. 8).

Similarly, the staining performance of anti Whitlow-PE clone MB524-B07 was superior over the two commercially available anti Whitlow-PE conjugate clone E3U7Q and clone 1B4A1 (see Fig. 9A and Fig. 9B).

### Description of the sequences of the sequence protocol

SEQ ID NO:1: Whitlow linker
SEQ ID NO:2: VH of MB524-B07
SEQ ID NO:3: VL of MB524-B07
SEQ ID NO:4: scFv of MB524-B07
SEQ ID NO:5: MB524-A08_scFv
SEQ ID NO:6: MB524-A02_scFv
SEQ ID NO:7: MB524-A07_scFv
SEQ ID NO:8: MB524-A10_scFv
SEQ ID NO:9: MB524-C06_scFv
SEQ ID NO:10: MB524-D03_scFv
SEQ ID NO:11: MB524-D05_scFv
SEQ ID NO:12: MB524-E09_scFv
SEQ ID NO:13: MB524-G03_scFv
SEQ ID NO:14: MBS525-A01 scFv
SEQ ID NO:15: MB525-A02_scFv
SEQ ID NO:16: MB525-A05_scFv
SEQ ID NO:17: MB525-A11_scFv
SEQ ID NO:18: MB525-B11_scFv
SEQ ID NO:19: MB525-C05_scFv
SEQ ID NO:20: MB525-F04_scFv
SEQ ID NO:21: MB525-G06_scFv
SEQ ID NO:22: MB525-G07_scFv
SEQ ID NO:23: MB525-G09_scFv
SEQ ID NO:24: MB525-G10_scFv
SEQ ID NO:25: MB525-H09_scFv
SEQ ID NO:26: MBS525-H11 scFv
SEQ ID NO:27: MB526-A01_scFv
SEQ ID NO:28: MB526-A07_scFv
SEQ ID NO:29: MB526-C07_scFv
SEQ ID NO:30: MB526-D12_scFv
SEQ ID NO:31: MB526-E03_scFv
SEQ ID NO:32: MB526-G12_scFv
SEQ ID NO:33: MB527-C07_scFv
SEQ ID NO:34: MB527-E01_scFv
SEQ ID NO:35: MB527-E07_scFv
SEQ ID NO:36: MB527-F07_scFv
SEQ ID NO:37: MB527-G03_scFv
SEQ ID NO:38: Whitlow_1
SEQ ID NO:39: Whitlow_2
SEQ ID NO:40: Whitlow_scFv
SEQ ID NO:41: CD2019 binder: CD20 VH (GGGGS)3 - CD20 VL -(GGGGS)5 -CD19 VL-Whitlow linker- CD19 VH
SEQ ID NO:42: VH of clone 16
SEQ ID NO:43: VLof clone 16

## Claims

1. An antibody or antigen binding fragment thereof specific for the amino acid sequence set forth in SEQ ID NO: 1, wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.

2. The antibody or antigen binding fragment thereof of claim 1 wherein said antibody or antigen binding fragment thereof is a scFv comprising SEQ ID NO:4.

3. The antibody or antigen binding fragment thereof of claim 1 or 2, wherein said antibody or antigen binding fragment thereof is coupled to a detection moiety.

4. The antibody or antigen binding fragment thereof of claim 3, wherein said detection moiety is a chromophore, a fluorescent moiety, a phosphorescent moiety, a luminescent moiety, a light absorbing moiety, a radioactive moiety, a transition metal isotope mass tag moiety, or a magnetic particle.

5. A kit comprising an antibody or antigen binding fragment thereof according to claim 3 or 4.

6. An in vitro method for detection, analysis and or separation (isolation) of immune cells in a sample, wherein said immune cells express a chimeric antigen receptor (CAR) comprising an extracellular antigen binding domain comprising the amino acid sequence set forth in SEQ ID NO: 1, the method comprising
i) contacting the CAR expressing immune cells of said sample with an antibody or antigen binding fragment thereof specific for the amino acid sequence set forth in SEQ ID NO: 1, wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3,
ii) determining, analyzing and/or separating (isolating) the CAR expressing immune cells of said sample.

7. The method of claim 6, wherein said antibody or antigen binding fragment thereof is coupled to a detection moiety.

8. The method of claim 7, wherein said detection moiety is a chromophore, a fluorescent moiety, a phosphorescent moiety, a luminescent moiety, a light absorbing moiety, a radioactive moiety, a transition metal isotope mass tag moiety, or a magnetic particle.

9. The method of claims 6 to 8, wherein said amino acid sequence set forth in SEQ ID NO: 1 is between a VL and a VH of the antigen binding domain of said CAR.

10. The method of claim 9, wherein said antigen binding domain of said CAR is a scFv.

11. The method of claims 6 to 10, wherein said immune cells are T cell, NK cells or HSCs.

12. The use of an antibody or antigen binding fragment thereof for detection, analysis and/or separation of immune cells in a sample, wherein said immune cells express a chimeric antigen receptor (CAR) comprising an extracellular antigen binding domain comprising the amino acid sequence set forth in SEQ ID NO:1, wherein said antibody or antigen binding fragment thereof is specific for the amino acid sequence set forth in SEQ ID NO:1, wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.

13. A nucleic acid encoding an antibody or antigen binding fragment thereof specific for the amino acid sequence set forth in SEQ ID NO:1, wherein said antibody or antigen binding fragment thereof comprises
a) a heavy chain variable region of an antibody (VH) comprising SEQ ID NO:2, and
b) a light chain variable region of an antibody (VL) comprising SEQ ID NO:3.
